# EUROPEAN PATENT SPECIFICATION

(11) **EP 1 417 301 B1**
(45) Date of publication and mention of the grant of the patent: **02.11.2006**
(21) Application number: 02767141.1
(22) Date of filing: 06.08.2002
(51) Int. Cl.: C12N 9/00, C12N 9/24, C12N 9/96

(54) **CARBOHYDRATES AND POLYOLS FOR DISSOLVING PROTEIN CRYSTALS**
KOHLHYDRATE UND POLYOLE ZUM LÖSEN DER PROTEINKRISTALLE
HYDRATES DE CARBONES ET POLYOLS PERMETTANT DE DISSOUDRE LES CRISTAUX DE PROTEINES

(30) Priority: 07.08.2001 US 310717 P
(43) Date of publication of application: 12.05.2004
(73) Proprietor: Novozymes A/S, 2880 Bagsvaerd (DK); Novozymes North America, Inc., New York, NY 10110 (US)
(72) Inventor: KAASGAARD, Svend, DK-2860 Soeborg (DK); RANE, Kishore, Raleigh, NC 27605 (US); HESS, Michael, Raleigh, NC 27615 (US); CHAMBERS, Thomas, Jr., Knightdale, NC 27545 (US)
(74) Representative: Kofoed, Gertrud Sonne
(86) International application number: PCT/DK2002/000525
(87) International publication number: WO 2003/014339

(56) References cited:
- EP-A- 0 201 017
- EP-A- 0 410 207
- BE-A- 657 602
- US-A- 4 588 691

## Description

### TECHNICAL FIELD

The present invention relates to a method of avoiding formation of protein crystals/amorphous precipitate during recovery processing.

### BACKGROUND ART

Some proteins are fermented in yields that are above their solubility limit, meaning that they are present in the culture broth in a partly precipitated form. The precipitate may be crystals or an amorphous precipitate.

Some proteins may be fermented in yields that are below their solubility limit, but the protein of interest is then concentrated during recovery processing to a concentration above its solubility limit, meaning that the protein becomes partly crystallised.

Furthermore, there is a trend towards operating with enzyme solutions at higher concentrations in order to save water in the downstream process and also due to customers requiring high strength products.

It is thus necessary to take special precautions in order to keep the protein in solution or bring the protein into solution again if already present in a precipitated form; if not the recovery yield of the protein will decrease.

The purpose of this invention is therefore to provide a simple and efficient solution to the above described problem.

### SUMMARY OF THE INVENTION

It has surprisingly been found that precipitation or crystallisation of a protein in a culture solution may be prevented in a simple and efficient way by adding a carbohydrate and/or a polyol to the culture solution during recovery processing, whereby the effective solubility of the protein is increased. In particular we claim:
a method for recovering a protein of interest from a culture solution comprising a recovery step in which step the protein of interest is in solution but above its solubility limit, comprising adding a carbohydrate, and/or a polyol and/or a derivative thereof, to the culture solution prior to, or immediately after, said recovery step.

### DETAILED DISCLOSURE OF THE INVENTION

The present invention deals with a new and surprisingly effective way of avoiding or reducing formation of protein crystals or precipitates.

It has surprisingly been found that crystallisation/precipitation of a protein can be prevented by adding a carbohydrate and/or a polyol and/or a derivative thereof to the culture solution during recovery processing, whereby crystallization/precipitation of the protein is reduced.

By avoiding formation of protein crystals/precipitates during recovery processing, a much more simple recovery process can be used resulting in higher yields.

In a preferred embodiment, the method is applied to enzymes, in particular to hydrolases (class EC 3 according to Enzyme Nomenclature; Recommendations of the Nomenclature Committee of the International Union of Biochemistry).

In a particular preferred embodiment the following hydrolases are preferred:
Proteases: Suitable proteases include those of animal, vegetable or microbial origin. Microbial origin is preferred. Chemically modified or protein engineered mutants are included (including substitutions, insertions, and/or deletions). The protease may be an acid protease, a serine protease or a metallo protease, preferably an alkaline microbial protease or a trypsin-like protease. Examples of alkaline proteases are subtilisins, especially those derived from *Bacillus,* e.g., subtilisin Novo, subtilisin Carlsberg, subtilisin 309, subtilisin 147 and subtilisin 168 (described in WO 89/06279). Examples of trypsin-like proteases are trypsin (e.g. of porcine or bovine origin) and the *Fusarium* protease described in WO 89/06270 and WO 94/25583.

Examples of useful proteases are the variants described in WO 92/19729, WO 98/20115, WO 98/20116, and WO 98/34946, especially the variants with substitutions in one or more of the following positions: 27, 36, 57, 76, 87, 97, 101, 104, 120, 123, 167, 170, 194, 206, 218, 222, 224, 235 and 274.

Preferred commercially available protease enzymes include Alcalase^{™}, Savinase^{™}, Primase^{™}, Duralase^{™}, Esperase^{™}, Relase^{™} and Kannase^{™} (Novozymes A/S), Maxatase^{™}, Maxacal^{™}, Maxapem^{™}, Properase^{™}, Purafect^{™}, Purafect OxP^{™}, FN2^{™}, and FN3™ (Genencor International Inc.).
Lipases: Suitable lipases include those of bacterial or fungal origin. Chemically modified or protein engineered mutants are included (including substitutions, insertions, and/or deletions). Examples of useful lipases include lipases from *Humicola* (synonym *Thermomyces*)*,* e.g. from *H. lanuginosa* (*T. lanuginosus*) as described in EP 258 068 and EP 305 216 or from *H. insolens* as described in WO 96/13580, a *Pseudomonas* lipase, e.g. from *P. alcaligenes* or *P. pseudoalcaligenes* (EP 218 272), *P. cepacia* (EP 331 376), *P. stutzeri* (GB 1,372,034), *P. fluorescens, Pseudomonas sp.* strain SD 705 (WO 95/06720 and WO 96/27002), *P. wisconsinensis* (WO 96/12012), a *Bacillus* lipase, e.g. from *B. subtilis* (Dartois et al. (1993), Biochemica et Biophysica Acta, 1131, 253-360), *B. stearothermophilus* (JP 64/744992) or *B. pumilus* (WO 91/16422).

Other examples are lipase variants such as those described in WO 92/05249, WO 94/01541, EP 407 225, EP 260 105, WO 95/35381, WO 96/00292, WO 95/30744, WO 94/25578, WO 95/14783, WO 95/22615, WO 97/04079 and WO 97/07202.

Preferred commercially available lipase enzymes include Lipolase^{™}, Lipolase Ultra^{™} and Lipex^{™} (Novozymes A/S).
Amylases: Suitable amylases (α and/or β) include those of bacterial or fungal origin. Chemically modified or protein engineered mutants are included (including substitutions, insertions, and/or deletions). Amylases include, for example, α-amylases obtained from *Bacillus,* e.g. a special strain of *B. licheniformis,* described in more detail in GB 1,296,839.

Examples of useful amylases are the variants described in WO 94/02597, WO 94/18314, WO 96/23873, and WO 97/43424, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.

Commercially available amylases are Duramyl^{™}, Termamyl^{™}, Fungamyl^{™}, Natalase^{™}, Termamyl LC^{™}, Termamyl SC^{™}, Liquizyme-X^{™} and BAN^{™} (Novozymes A/S), Rapidase^{™} and Purastar^{™} (from Genencor International Inc.).
Cellulases: Suitable cellulases include those of bacterial or fungal origin. Chemically modified or protein engineered mutants are included (including substitutions, insertions, and/or deletions). Suitable cellulases include cellulases from the genera *Bacillus, Pseudomonas, Humicola, Fusarium, Thielavia, Acremonium,* e.g. the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and PCT/DK98/00299.

Commercially available cellulases include Celluzyme^{™}, and Carezyme^{™} (Novozymes A/S), Clazinase^{™}, and Puradax HA^{™} (Genencor International Inc.), and KAC-500(B)^{™} (Kao Corporation).

### Oxidoreductases

Oxidoreductases that may be treated according to the invention include peroxidases, and oxidases such as laccases, and catalases (EC 1.11.1.6).

### Peroxidases

An enzyme exhibiting peroxidase activity may be any peroxidase enzyme comprised by the enzyme classification (EC 1.11.1.7), or any fragment derived therefrom, exhibiting peroxidase activity.

Preferably, the peroxidase employed in the method of the invention is producible by microorganisms such as fungi or bacteria. Some preferred fungi include strains belonging to the subdivision Deuteromycotina, class Hyphomycetes, e.g., Fusarium, Humicola, Tricoderma, Myrothecium, Verticillum, Arthromyces, Caldariomyces, Ulocladium, Embellisia, Cladosporium or Dreschlera, in particular Fusarium oxysporum (DSM 2672), Humicola insolens, Trichoderma resii, Myrothecium verrucana (IFO 6113), Verticillum alboatrum, Verticillum dahlie, Arthromyces ramosus (FERM P-7754), Caldariomyces fumago, Ulocladium chartarum, Embellisia alli or Dreschlera halodes.

Other preferred fungi include strains belonging to the subdivision Basidiomycotina, class Basidiomycetes, e.g. Coprinus, Phanerochaete, Coriolus or Trametes, in particular Coprinus cinereus f. microsporus (IFO 8371), Coprinus macrorhizus, Phanerochaete chrysosporium (e.g. NA-12) or Trametes (previously called Polyporus), e.g. T. versicolor (e.g. PR4 28-A).

Further preferred fungi include strains belonging to the subdivision Zygomycotina, class Mycoraceae, e.g. Rhizopus or Mucor, in particular Mucor hiemalis.

Some preferred bacteria include strains of the order Actinomycetales, e.g., Streptomyces spheroides (ATTC 23965), Streptomyces thermoviolaceus (IFO 12382) or Streptoverticillum verticillium ssp. verticillium.

Other preferred bacteria include Bacillus pumilus (ATCC 12905), Bacillus stearothermophilus, Rhodobacter sphaeroides, Rhodomonas palustri, Streptococcus lactis, Pseudomonas purrocinia (ATCC 15958) or Pseudomonas fluorescens (NRRL B-11).

Further preferred bacteria include strains belonging to Myxococcus, e.g., M. virescens.

Particularly, a recombinantly produced peroxidase is preferred, e.g., a peroxidase derived from a Coprinus sp., in particular C. macrorhizus or C. cinereus according to WO 92/16634, or a variant thereof, e.g., a variant as described in WO 93/24618 and WO 95/10602.

### Laccases and Laccase Related Enzymes

In the context of this invention, laccases and laccase related enzymes contemplate any laccase enzyme comprised by the enzyme classification (EC 1.10.3.2), any chatechol oxidase enzyme comprised by the enzyme classification (EC 1.10.3.1), any bilirubin oxidase enzyme comprised by the enzyme classification (EC 1.3.3.5) or any monophenol monooxygenase enzyme comprised by the enzyme classification (EC 1.14.18.1).

The microbial laccase enzyme may be derived from bacteria or fungi (including filamentous fungi and yeasts) and suitable examples include a laccase derivable from a strain of Aspergillus, Neurosaora, e.g., N. crassa, Podospora, Botrytis, Collybia, Fomes, Lentinus, Pleurotus, Trametes, e.g., T. villosa and T. versicolor, Rhizoctonia, e.g., R. solani, Coprinus, e.g. C. plicatilis and C. cinereus, Psatyrella, Myceliophthora, e.g. M. thermophila, Schytalidium, Polyporus, e.g., P. pinsitus, Phlebia, e.g., P. radita (WO 92/01046), or Coriolus, e.g., C. hirsutus (JP 2-238885), in particular laccases obtainable from Trametes, Myceliophthora*,* Schytalidium or Polyporus.

Other preferred hydrolases are glycosidases including Mannaway^{™}. Other preferred enzymes are transferases, lyases, isomerases, and ligases.

### Recovery processing

The protein of interest may be fermented by methods known in the art, e.g., as described in US 3,723,250 for the fermentation of the protease, Savinase^{™}.

A process for the recovery of a protein of interest from a fermentation broth will typically (but is not limited to) involve some or all of the following steps:
1) pre-treatment of broth
2) removal of cells and other solid material from broth (primary separation)
3) filtration
4) concentration
5) filtration
6) stabilization and standardization.

In the pre-treatment step, cells and solid material are flocculated and some soluble components are precipitated. This step may be carried out as described in WO 96/38469. The removal of cells and other solid material may be carried out by centrifugation or filtration. The filtration step may be included in order to improve the clarity of the protein solution and/or to remove microbial cells. The concentration step will typically involve an ultra-filtration step or an evaporation of water at reduced pressure. During the concentration, solid material may be formed which can be removed by further filtrations. Finally, the protein product is stabilized and standardized to the desired concentration as known in the art. Apart from the unit operations listed above, a number of other recovery procedures and steps may be applied, e.g., pH-adjustments, variation in temperature, treatment of protein solution with active carbon, and use of various adsorbents, e.g. to remove undesired compounds such as side-activities and coloured compounds.

Depending on the protein of interest, the fermentation conditions and the fermentation yields, the protein may be present in a precipitated form in the culture broth or may precipitate during the recovery process. In the latter case, the precipitation may be initiated by changes in pH, temperatures, or salt-concentration, or by increasing concentration of the protein it-self.

We have now found that addition of polyol and/or carbohydrates to the protein containing solution may prevent precipitation of the protein or help dissolving already precipitated protein. The polyol and/or carbohydrate must be added immediately prior to the recovery step where the precipitate is typically formed

If the protein of interest is precipitated already before harvest of the culture broth, the polyol and/or carbohydrate should be added before the primary separation. If the protein of interest is precipitating during or after the concentration step, the polyol and/or carbohydrate may be added either prior to the concentration step or after the concentration step but before e.g. a filtration step downstream. In some cases, precipitation of the protein during the concentration step is advantageous as the protein is often more stable in a precipitated form. This could be the case e.g. during concentration by evaporation at reduced pressure if the protein containing solution is heated up in the process to facilitate removal of water. Here the polyol and/or carbohydrate could be added after the concentration step. The precipitation may be spontaneous and uncontrolled or preferably a controlled process in which the protein is precipitated as crystals. Crystallization may be carried out in a number of ways (e.g. as described in WO 91/09943 or WO 94/22903). It is shown in Example 2 and 3 that sucrose and sorbitol are especially advantageous for re-dissolving crystals/precipitates. In other cases, the presence of enzyme in a partly precipitated form during evaporation is undesirable. Here the enzyme may be kept in solution by addition of a polyol and/or a carbohydrate in a low concentration, typically 1-10 % w/w prior to the concentration step. Both the concentration of the protein and the polyol and/or the carbohydrate will then increase. The solubility limit is thus increased during the processing step.

In other circumstances, precipitates are undesired in a recovery unit operation (e.g. due to blocking of filtration membranes, or wear and tear of the membranes). Here the polyol and/or carbohydrate could be added prior to the unit operation whereby the formation of precipitate is avoided.

Accordingly, we claim the following embodiments in the present invention:
a: A method for recovering a protein of interest from a culture solution comprising: adding a polyol having the formula CₙH₂ₙ₊₂Oₙ, wherein n is from 4 to 8, and/or a carbohydrate, and/or a derivative thereof, to the culture solution before evaporation.
b: A method for recovering a protein of interest from a culture solution comprising: adding a polyol having the formula CₙH₂ₙ₊₂Oₙ, wherein n is from 4 to 8, and/or a carbohydrate, and/or a derivative thereof, to the culture solution before or immediately after a recovery concentration unit step.
c: A method for recovering a protein of interest from a culture solution comprising: adding a polyol having the formula CₙH₂ₙ₊₂Oₙ, wherein n is from 4 to 8, and/or a carbohydrate, and/or a derivative thereof, to the culture solution after ultrafiltration and before granulation.

In some cases the solubility of the protein can be further increased in the recovery process by running part of or the entire process at high pH (typically 9 < pH < 12), or at low pH (below pH 6). The solubility of the protein can also be influenced by the presence of salts and by temperature. The effect on solubility will depend on the characteristics of the protein.

### Solubility limit

As mentioned above the solubility of a protein is controlled by a number of factors such as temperature, pH, the presence of salts, other proteins and impurities. A phase diagram is a description of the solubility of a protein as a function of e.g. salt concentration, pH, temperature or amount of polyol. Such a phase diagram is commonly described as containing at least four different zones (see e.g. M. Riès-Kautt and A. Ducruix in "Crystallization of Nucleic Acids and Proteins. A practical approach", eds.: A. Ducruix and R. Giegé, IRL Press at Oxford University Press (1992) p. 201). Starting from an aqueous suspension of a precipitated protein at a given set of conditions (pH, temperature, salts etc), the solubility is defined as the concentration of protein in the aqueous phase when this system composed of the protein of interest in both soluble and precipitated form has reached equilibrium.

If the protein concentration is lower than its solubility, i.e. the solution is undersaturated; all the protein will be on soluble form. In general however, if the total protein concentration is higher than the solubility of the protein, the solution is supersaturated with respect to the protein. This supersaturated zone can be subdivided into three zones: (a) the precipitation zone where the excess protein precipitates from the solution in an amorphous state; (b) a nucleation zone where protein crystal nuclei can form and excess protein will precipitate in crystalline form and (c) a metastable zone where protein nuclei crystals cannot form but crystals already present can grow. The crystals will continue to grow until the soluble protein concentration reaches the solubility limit. If starting from a protein solution where the protein is fully dissolved, it is thus possible to increase the protein concentration above the solubility limit without any protein precipitates are formed, if the protein concentration is kept below the concentration separating the nucleation zone and the metastable zone. As the crystallization of proteins most often is a rather slow process, it is often even possible - within a short time frame - to enter the nucleation zone during a concentration step without precipitates being formed, i.e. it is thus possible to maintain the concentrated protein solution in a state free of protein crystals or protein precipitates for seconds, minutes or even hours even though the protein concentration is within the nucleation zone.

The effective solubility is the concentration of soluble protein in a solution, the effective solubility can be higher than the solubility of the protein at a given set of conditions when the solution is supersaturated.

In EP 0 201 017, a process is described for first precipitating an enzyme by addition of a salt, then isolating the enzyme cake and finally dissolving the enzyme by contacting the cake with a polyol. The polyols contemplated in EP 0 201 017 comprise low molecular weight polyethylene glycol and the C₂ through C₈ alcohols having at least two OH groups. According to the invention polyols with more than two OH groups, including glycerol, can be employed but it is stated that alcohols having only two OH groups are preferred, especially when they are present on adjacent carbon atoms in the chain.

However, the precipitation and following isolation of the precipitate is often time consuming especially if the proteins are to be precipitated as crystals to increase purity. Quite significant yield losses are frequently seen when operated in large scale both due to poor precipitation yields and to physical losses. Although some of the salt used to precipitate the protein may be recycled, it adds an additional cost to the process both in form of the recycling process itself and the additional expenses in the waste water treatment (due to the added salts). Furthermore, special measures have to be taken to remove the salt from the enzyme product, when the salt is undesired in the final product. On the other hand, precipitation of protein is a way to provide protein material in sufficient high concentrations for further formulation (either in liquid or solid form). High concentrations of e.g. monopropylene glycol are needed to dissolve the enzyme as the concentration of the enzyme cannot exceed the solubility limit when starting from a solution consisting of both soluble and precipitated enzyme, i.e. a supersaturated state cannot be reached when just adding a polyol like MPG to a cake of salt precipitated protein.

However, according to the present invention the salt precipitation step can be eliminated and a high strength concentrate can still be obtained without having the protein of interest in precipitated form. Here the protein is concentrated by e.g. ultra-filtration or evaporation. The protein of interest may be concentrated to a concentration which is within the metastable zone or even within the nucleation zone.

The out-let from the concentration unit may be led directly into a tank containing a carbohydrate or a polyol. Alternatively the carbohydrate and/or polyol may be added continuously to the process stream containing the concentrate coming out of the concentration unit. When the carbohydrate and/or the polyol is added to the concentrate before the first crystals are formed it is possible to obtain a concentrate of a high protein concentration without having the protein in precipitated or crystalline form. The carbohydrate and/or polyol should be added in an amount sufficient to bring the protein concentrate into the undersaturated or metastable zone. If operating within the metastable zone a crystallization or a precipitation may be initiated by applying mechanical stress or adding fine particles to the solution. If this is the case care should be taken when handling the product in subsequent downstream processes.

However, as the protein concentration approaches the solubility line, the solution becomes more and more stable with respect to its ability to precipitate and/or crystallize.

Apart from adding carbohydrate and/or polyol to the concentrate, other factors such as pH, temperature, addition of salts can be used to further increase the solubility of the protein of interest. The effective solubility will increase when the carbohydrate and/or polyol is added. Much higher soluble protein concentrations can thus be reached at lower polyol concentrations than it is possible if the teachings of EP 0 201 017 are followed. In a preferred mode of the invention, the concentration step is performed in a continous mode. Hereby, the time of concentrations can be reduced and it is thus possible to reach even higher concentrations before the protein starts to precipitate, if the out-let from the concentration unit is led directly into the tank containing the carbohydrate and/or polyol or the carbohydrate and/or polyol is added continuously to the process stream containing the concentrate coming out of the concentration unit as described above.

In a preferred mode the concentration of carbohydrate or polyol after addition to the protein solution is kept above 2% w/w during the process. Preferred polyols are MPG, glycerol or sorbitol or a mixture hereof. Preferred mono-, di- and poly-saccharides are sucrose, glucose, fructose, maltose, lactose, galactose, lactose, maltose, isomaltose, trehalose, maltulose, sucrose, dextrins, limit dextrins, cyclodextrins, or amylopectins.

It may in some cases be an advantage also to add salts to the process stream containing the concentrate coming out of the concentration unit, e.g. a combination of salt such as NaCl or KCI and a carbohydrate such as glucose, maltose or sucrose and/or a polyol such as sorbitol.

Accordingly we describe in the present invention:
A method for recovering a protein of interest from a culture solution comprising a recovery step in which step the protein of interest is in solution but above its solubility limit, comprising adding a carbohydrate, and/or a polyol and/or a derivative thereof, to the culture solution prior to, or immediately after, said recovery step.

When producing liquid products, a filtration step is often implemented after the concentration step, even though the presence of precipitated protein is avoided during the process to obtain a higher clarity of the product or to reduce the number of microorganisms in the product. As the viscosity of a carbohydrate or polyol solution increases with concentration, it is important to keep the concentration of the carbohydrate or polyol as low as possible in order to ensure high fluxes during downstream filtrations.

The establishment of a phase diagram describing the solubility of a given protein is very difficult, especially identifying the borderlines between the different zones in the supersaturated state. One of the reasons why it is difficult is that the formation of crystal nuclei is a stochastic event. A protein solution in the nucleation zone can thus remain clear without any crystals being formed for years before stable crystal nuclei are formed which can grow into crystals, also the time before a given system reaches equilibrium at a given set of conditions can be practically indefinitely.

However, in this invention we define the solubility of a protein as the concentration of the protein in the aqueous phase after stirring the solution containing the protein of interest in both soluble and precipitated form gently for 24 hours at constant conditions i.e. keeping the pH, temperature, and the concentration of all components in the solution constant as well as other factors which may influence the solubility of the protein. Information on which factors can influence protein solubility can be found in the literature, see e.g. "Crystallization of Nucleic Acids and Proteins. A practical approach", eds.: A. Ducruix and R. Giegé, IRL Press at Oxford University Press (1992) and "Protein Crystallization. Techniques, Strategies, and Tips", Ed.: T.M. Bergfors, International University Line (1999). The concentration between the nucleation zone and the metastable zone is determined by the following procedure:
A: Concentrating the protein solution stepwise by ultrafiltration at the desired pH, temperature, concentration of salts, carbohydrates and polyols, starting from a clear solution of the protein.
B: At certain intervals an aliquot of the concentrate is removed and quickly filtered through a 0.22 µm filter (the concentration of the protein in the aqueous phase in this filtered sample is referred to as the starting concentration).
C: The filtered sample is stirred gently for up to 48 hours and it is inspected frequently. If an amorphous precipitate is formed, the starting concentration is within the precipitation zone. This precipitation will often be a fast process. If crystals forms spontaneously in the filtered sample, the starting concentration is within the nucleation zone; if no crystals are formed the starting concentration is below the nucleation zone.
D: If no crystals or precipitate in the filtered sample from (C) is formed, a few crystals of the protein of interest (seeding) is added and the samples is stirred for further 10 hours.
E: At regular intervals new aliquots are taken from the sample taken from (D) and potential changes in crystal number and size is determined. The crystal size can be evaluated by a number of ways known within the art, e.g. by microscopy and image analysis. If the added crystals are decreasing in size the solution is undersaturated, if the crystals size increases the starting protein solution is in the metastable zone. If new crystals are being formed the starting protein solution is in the nucleation zone. If the added crystals neither increase in number or decrease in size, the protein concentration in the aqueous phase is at the solubility limit. By narrowing down the time between taking samples during the ultrafiltration, a phase diagram can be constructed which is useful for practical purposes.

It is important to keep the temperature constant during the entire experiment. Furthermore, the test should be performed under sterile conditions. Alternatively, an antimicrobial agent such as Rodalon^{™}, Proxel LV^{™} or sodium azide can be added if it does not affect the solubility of the protein of interest.

### Carbohydrates

Any carbohydrate as defined in John F. Robyt: Essentials of Carbohydrate Chemistry, p. 2 (1998): "the modern definition of a carbohydrate is that it is a polyhydroxy aldehyde or ketone, or a compound that can be derived from them by any of several means including (1) reduction to give sugar alcohols, (2) oxidation to give sugar acids; (3) substitution of one or more of the hydroxyl groups by various chemical groups, for example, hydrogen [H] may be substituted to give deoxysugars, and amino group [NH₂ or acetyl-NH] may be substituted to give amino sugars; (4) derivatization of the hydroxyl groups by various moieties, for example, phosphoric acid to give phosphor sugars, or sulphuric acid to give sulfo sugars, or reaction of the hydroxyl groups with alcohols to give saccharides, oligosaccharides, and polysaccharides."

The carbohydrates contemplated in this invention are having four or more carbon atoms; preferably from 4 to 12 carbon atoms; in particular from 4 to 6 carbon atoms.

According to the present invention a polyhydroxy aldehyde or a ketone is preferred.

In particular a mono-saccharide selected from the group consisting of glucose, fructose, mannose and galactose is preferred.

Of the substituted sugars, methyl glycosides, N-acetyl-glucosamine, N-acetylgalactosamine and their de-acetylated forms are preferred.

In another preferred embodiment a di-saccharide selected from the group consisting of lactose, maltose, isomaltose, trehalose, maltulose, and sucrose is preferred.

Among the oligosaccharides, dextrins, limit dextrins, cyclodextrins, and amylopectins are preferred. The sugar acids may include both "uronic" and "onic" acids such as gluconic acid, glucuronic acid and galacturonic acid.

In a particular embodiment of the invention the carbohydrate is added to the culture solution at an amount of 0.1-75% w/w; preferably at an amount of 0.1-50% w/w; more preferably at an amount of 0.1-40% w/w; more preferably at an amount of 0.1-25% w/w; more preferably at an amount of 0.1-10% w/w; more preferably at an amount of 0.1-5% w/w; in particular at an amount of 0.5-5% w/w.

In some cases it may be an advantage to use a mixture of a carbohydrate and a salt; e.g. a mixture of sucrose and NaCl or KCI.

### Polyols

Any polyol may be used. However, a polyol selected from the group derived from the carbohydrates having at least four carbon atoms are preferred. These have the general formula CₙH₂ₙ₊₂Oₙ, wherein n is from 4 to 8 carbon atoms; in particular n is from 4 to 6 carbon atoms.

They include but are not limited to sorbitol, mannitol, erythritol, ribitol, and xylitol. In certain aspects of the invention glycerol and monopropylene glycol (MPG) may also be used.

In a particular embodiment of the invention the polyol is added to the culture solution at an amount of 0.1-80% w/w; preferably at an amount of 0.1-60% w/w; more preferably at an amount of 0.1-40% w/w; more preferably at an amount of 0.1-20% w/w; more preferably at an amount of 0.1-10% w/w; more preferably at an amount of 0.1-5% w/w; in particular at an amount of 0.5-5% w/w.

In some cases it may be an advantage to use a mixture of two or more polyols, e.g. glycerol and monopropylene glycol, or a mixture of a polyol and a carbohydrate, e.g. glucose and sorbitol.

In some cases it may be an advantage to use a mixture of a polyol and a salt; e.g. a mixture of sorbitol and NaCl or KCI.

Preferably the carbohydrate and/or polyol of choice is the one also used for stabilizing the protein in the final product.

### Derivatives

Derivatives that may be used according to the invention include methyl glycosides, glucoronic acids, amino sugars, or N-acetyl glucosamines.

### Adjustment of pH

The pH of the culture solution to which the carbohydrate and/or the polyol and/or the derivative thereof has been added, may be adjusted in order to find the optimum wherein the effective solubility of the protein of interest is maximized and at the same time the protein is stable.

A way of finding this optimum is to run a trial, typically starting at pH 11, then perform the test at pH 10, then perform the test at pH 9, then at pH 8, then at pH 7, and so on down to pH 3, and then, if it is found, e.g., that the optimum is between pH 4 and pH 5, then do a trial within this range whereby the optimum pH is found; the optimum will normally be in the range between pH 4 and pH 11.

For adjustment of pH virtually any acid or base can be used. The acid may be inorganic or organic. Some examples are hydrochloric acid, sulphuric acid, sulphurous acid, nitrous acid, phosphoric acid, acetic acid, citric acid, and formic acid. Preferred acids are phosphoric acid, formic acid, citric acid, and acetic acid. Preferred bases are sodium hydroxide, potassium hydroxide, calcium hydroxide, and ammonium hydroxide, in particular sodium hydroxide.

### Adjustment of temperature

The temperature of the culture solution to which the carbohydrate and/or the polyol and/or the derivative thereof has been added, may be adjusted in order to find the optimum wherein the effective solubility of the protein of interest is maximized and at the same time the protein is stable.

A way of finding this optimum is to run a trial, typically starting at 5°C, then perform the test at 10°C, then at 20°C, then at 30°C, then at 40°C, then at 50°C, then at 60°C, and then at 70°C; and then, if it is found, e.g., that the optimum is between 30°C and 40°C, then do a trial within this range whereby the optimum temperature is found. It is to be noted that the solubility of some enzymes at certain conditions will increase with decreasing temperature.

### Salts

In order to further improve the recovery processing one or more salts may be added in order to stabilize the protein or increase the solubility.

Preferred salts are: magnesium, sodium, potassium, ammonium or calcium salts of chloride, citrate, acetate, formiate or sulphate, e.g., MgCl₂, CaCl₂, NaCl, KCI, NH₄Cl, MgSO₄, Na₂SO₄, K₂SO₄, or (NH₄)₂SO₄.

In a particular embodiment of the invention the salt is added to the culture solution at a concentration of 0.02-10 moles per liter, preferably at a concentration of 0.02-5 moles per liter, more preferably at a concentration of 0.02-1.2 moles per liter, more preferably at a concentration of 0.04-1.0 moles per liter, more preferably at a concentration of 0.04-0.7 moles per liter, most preferably at a concentration of 0.04-0.5 moles per liter.

The salt according to the invention may be added in one step, or it may be added in more than one step; normally the recovery processing will perform satisfactorily when the salt is added in one step.

The invention is further illustrated in the following examples, which are not intended to be in any way limiting to the scope of the invention as claimed.

### EXAMPLE 1 (comparative)

### Addition of sugar/polyol to avoid crystallization/precipitation during downstream processing

Sorbitol (1.5% w/w) was added to the non-crystallized or non-precipitated protease enzyme solution prior to evaporation. The protease enzyme solution was concentrated from 20 to 38 RI. RI (refractive index) was measured at a B+S RFM80 digital refractometer.

The control concentrated solution had significant crystallization whereas the sorbitol containing concentrated protease solution had no crystallization. Crystallization was measured as % v/v spindown (centrifuged at 2000 rpm for 10 min). Control sample had 55.5% spindown whereas the sorbitol containing sample had no spindown.

This inhibition of crystallization and/or amorphous precipitation is very important. Typical downstream processes involve filtration steps to increase the clarity and/or to remove microorganism. If the product is crystallized or amorphous precipitated, then there is significant loss in the product yields.

Addition of sorbitol and/or sucrose (up to 5%) has resulted in 5% increased downstream processing yields.

### EXAMPLE 2 (comparative)

### Addition of sugar and/or polyol to dissolve the enzyme crystals/precipitate during downstream processing.

Addition of sugar and/or sorbitol with combination of heat treatment (up to 70°C) helps to dissolve the crystals and/or amorphous precipitate during the production of enzymes. Typically, spontaneous partial or complete crystallization and/or precipitation occurs in the enzyme solution at higher enzyme protein concentration or at higher ratio of enzyme protein to impurities. Such partial or complete crystallization/precipitation of enzyme protein causes physical loss or lower downstream production yields due to the losses occurring during the typical downstream operations such as filtration, centrifugation, or clarification. This example describes the situation when an amylase enzyme solution is crystallized/precipitated. The amylase enzyme solutions were crystallized in presence of different amounts of sugars and/or salts. All the solutions were crystallized partially or fully within 2-4 hours. To re-dissolve crystals/precipitate, solutions were heated up to 60°C. The amylase enzyme solutions containing sucrose and sucrose+salt (see Table 1) were fully dissolved. However, amylase enzyme solutions containing sucrose+salt re-crystallized upon cooling, whereas, amylase enzyme solutions containing only sucrose did not re-crystallize upon cooling back to room temperature.

**Table 1:**

| Sample | % Spin down after 24 hours | Crystals or precipitate dissolution at 60°C | Re-crystallization or precipitation of dissolved crystals or precipitate (after 2 days) |
|---|---|---|---|
| Control solution | 24% | No | NA |
| +5% sucrose | 16% | Yes | No |
| +10% Sucrose | 16% | Yes | No |
| +5% Sucrose + 5% NaCl | 17% | Yes | Yes |
| +10% Sucrose + 5% NaCl | 15% | Yes | Yes |

### EXAMPLE 3

### Addition of sorbitol and/or monopropylene glycol followed by pH adjustments.

Addition of sorbitol and/or monopropylene glycol with or without pH adjustments (up to pH 11.0) helps to dissolve the enzyme crystals and/or amorphous enzyme precipitate during the production of enzymes. Typically, spontaneous partial or complete crystallization and/or precipitation occurs in the enzyme solution at higher enzyme protein concentration or at higher ratio of enzyme protein to impurities. Such partial or complete crystallization/precipitation of enzyme protein causes physical loss or lower downstream production yields due to the losses occurring during the typical downstream operations such as filtration, centrifugation, or clarification. This example describes the situation when a freshly prepared concentrate of mannanase in 30% MPG is prepared. The solution remains clear for up to 2 hours, but after 24 hours the enzyme is present in a partly crystallized/precipitated form. In order to re-dissolve the crystals/precipitate, sorbitol and/or MPG or sucrose was added to the solution. The mannanase enzyme solutions containing sorbitol dissolved, (with 15% sorbitol or more) all mannanase enzyme crystals/precipitates.

Monopropylene glycol alone was not effective. It was effective (in high concentrations) when combined with a pH adjustment to 11.0. pH adjustment was not by itself effective because the mannanase enzyme crystals/precipitates reformed after the pH was decreased from pH 11.0. Clarity (NTU) in this example was measured at a Hach 2100AN Turbidimeter.

**Table 2**

| | Relative MPG | Relative Sorbitol | pH | Clarity | Volume of crystallized enzyme |
|---|---|---|---|---|---|
| | % | % | | NTU | % Spindown |
| Control (a) | 30 | 0 | 8.5 | + 200 | 20 |
| 5% Sorbitol (b) | 28.5 | 5 | 8.5 | + 200 | 12.5 |
| 10% Sorbitol (c) | 27 | 10 | 8.5 | + 200 | 1.5 |
| 15% Sorbitol (d) | 25.5 | 15 | 8.5 | 34 | 0 |
| 20% Sorbitol (e) | 24 | 20 | 8.5 | 26 | 0 |
| 25% Sorbitol (f) | 22.5 | 25 | 8.5 | 20 | . 0 |
| 25% MPG (g) | 47.5 | 0 | 8.5 | + 200 | 6 |
| pH 11 (h) | 30 | 0 | 8.5→11 | + 200 | 1.5 (20% after pH adjustment back to 8.5) |
| pH 11,25% MPG, 25% water (i) | 40 | 0 | 8.5→11 →8,5 | 45 | 0.5 |
| 15% MPG, 35% water (j) | 30 | 0 | 8.5 | + 200 | 3.4 |
| 15% MPG, 10% Sorbitol, 25% water (k) | 30 | 10 | 8.5 | 27 | 0 |

Table 2 also shows how the solubility of a protein (here a mannanase) increases with increasing sorbitol concentration (see experiments (a) to (f)).

Starting from a dilute culture solution of the mannanase, it is possible to concentrate it to the same concentration as used in experiment (a) to (k) without addition of MPG but with addition of sorbitol (25% w/w). Under these conditions no precipitates are formed after 24 hours, and the NTU is below 50.

### EXAMPLE 4

### Dissolve enzyme crystals/precipitate during downstream processing by addition of sugar and stirring.

After concentration of 1,4-a-D-glucan alpha-maltohydrolase (fermented as known in the art), the main part of the enzyme is present as crystals or a precipitate. The crystals/precipitate is observed as sludge in the concentrate. The amount of this sludge can be measured after centrifugation and is found to be in the range of 10 to 15 % (v/v).

After addition of 15% (w/w) sucrose and stirring for 30 minutes the enzyme crystals/precipitate is fully dissolved. No heat treatment is necessary to dissolve the enzyme crystals/precipitate, thus the sugar addition and stirring is done from 10 to 25°C.

The enzyme solution can now be further processed and finalized as either a stabilized liquid product or in a granulated form. For the liquid product, sucrose is also used as the stabilizer.

### EXAMPLE 5

### Use of MPG to avoid crystallization of alpha-amylase

Alpha-amylase from *B. licheniformis* (fermented as known in the art) was concentrated by ultrafiltration at pH 10.5 and at 30°C to approximately 100 g enzyme protein per kg. Two experiments (A and B) were run in parallel. One (A) was run as a control. To the other (B) 5% w/w MPG was added prior to ultrafiltration. After the concentration step both concentrates were left standing with slow stirring for 24 hours at 5°C. After the 24 hours a heavy crystalline precipitate had formed in sample A (10 % v/v sludge volume), whereas no precipitate could be observed in sample B. MPG is thus able to prevent formation of precipitate in the alpha-amylase concentrate.

Upon addition of a few alpha-amylase crystals (seeding) and further stirring at 5°C, 1-2 % v/v sludge volume was formed in the seeded sample B, showing that the concentrate with MPG was supersaturated.

## Claims

1. A method for recovering a protein of interest from a culture solution comprising a recovery step in which step the protein of interest is in solution but above its solubility limit, comprising adding a carbohydrate and/or a polyol to the culture solution immediately after said recovery step.

2. The method according to claim 1, wherein the protein is an enzyme.

3. The method according to claim 2, wherein the enzyme is selected from the group consisting of a hydrolase, a protease, an amylase, a cellulase, a lipase, an oxidoreductase, and a glycosidase.

4. The method according to claim 1, wherein the carbohydrate is selected from the group consisting of glucose, sucrose, glucose syrup, fructose, maltose, lactose, trehalose, oligosaccharides, limit dextrins, dextrins, starch, mannose, and galactose.

5. The method according to claim 1, wherein the polyol is selected from the group consisting of sorbitol, mannitol, erythritol, ribitol and xylitol.

6. The method according to claim 1, wherein the carbohydrate and/or the polyol is added immediately after a recovery concentration unit step.

7. The method according to claim 1, wherein the carbohydrate and/or the polyol is added immediately after ultrafiltration.

8. The method according to claim 1, wherein the carbohydrate is added in an amount of 0.1-75% (w/w).

9. The method according to claim 1, wherein the polyol is added in an amount of 0.1-80% (w/w).

10. The method according to claim 1, wherein the polyol is a mixture of two polyols.

11. The method according to claim 1, wherein pH is adjusted during recovery processing.

12. The method according to claim 1, wherein temperature is adjusted during recovery processing.

13. The method according to claim 1, wherein salts are added during recovery processing.

14. The method according to claim 13, wherein the salts are selected from the group consisting of magnesium, sodium, potassium, ammonium and calcium salts of chloride, citrate, acetate, formiate and sulphate.

## Patentansprüche

1. Verfahren zur Gewinnung eines Proteins von Interesse aus einer Kulturlösung, umfassend einen Gewinnungsschritt, in welchem Schritt sich das Protein von Interesse in Lösung, allerdings oberhalb seiner Löslichkeitsgrenze, befindet, umfassend das Zugeben eines Kohlehydrats und/oder eines Polyols zu der Kulturlösung unmittelbar nach dem Gewinnungsschritt.

2. Verfahren nach Anspruch 1, wobei das Protein ein Enzym ist.

3. Verfahren nach Anspruch 2, wobei das Enzym aus der Gruppe ausgewählt ist, bestehend aus einer Hydrolase, einer Protease, einer Amylase, einer Cellulase, einer Lipase, einer Oxidoreductase und einer Glycosidase.

4. Verfahren nach Anspruch 1, wobei das Kohlehydrat aus der Gruppe ausgewählt ist, bestehend aus Glucose, Saccharose, Glucosesirup, Fructose, Maltose, Lactose, Trehalose, Oligosacchariden, Grenzdextrinen, Dextrinen, Stärke, Mannose und Galactose.

5. Verfahren nach Anspruch 1, wobei das Polyol aus der Gruppe ausgewählt ist, bestehend aus Sorbit, Mannit, Erythrit, Ribitol und Xylit.

6. Verfahren nach Anspruch 1, wobei das Kohlehydrat und/oder das Polyol unmittelbar nach einem Gewinnungs-Konzentrierungs-Einheitsschritt zugesetzt wird.

7. Verfahren nach Anspruch 1, wobei das Kohlehydrat und/oder das Polyol unmittelbar nach einer Ultrafiltration zugesetzt wird.

8. Verfahren nach Anspruch 1, wobei das Kohlehydrat in einer Menge von 0,1-75 % (Gew./Gew.) zugesetzt wird.

9. Verfahren nach Anspruch 1, wobei das Polyol in einer Menge von 0,1-80 % (Gew./Gew.) zugesetzt wird.

10. Verfahren nach Anspruch 1, wobei das Polyol ein Gemisch von zwei Polyolen ist.

11. Verfahren nach Anspruch 1, wobei der pH während der Gewinnungsverarbeitung reguliert wird.

12. Verfahren nach Anspruch 1, wobei die Temperatur während der Gewinnungsverarbeitung reguliert wird.

13. Verfahren nach Anspruch 1, wobei die Salze während der Gewinnungsverarbeitung zugesetzt werden.

14. Verfahren nach Anspruch 13, wobei die Salze aus der Gruppe ausgewählt werden, bestehend aus Magnesium, Natrium, Kalium, Ammonium und Calciumsalzen von Chlorid, Citrat, Acetat, Formiat und Sulfat.

## Revendications

1. Méthode pour récupérer une protéine d'intérêt à partir d'une solution de culture comprenant une étape de récupération dans laquelle la protéine d'intérêt est dans la solution mais au-dessus de sa limite de solubilité, comprenant l'ajout d'un carbohydrate et/ou un polyol à la solution de culture immédiatement après ladite étape de récupération.

2. Méthode selon la revendication 1, dans laquelle la protéine est une enzyme.

3. Méthode selon la revendication 2, dans laquelle l'enzyme est choisie parmi le groupe constitué d'une hydrolase, une protéase, une amylase, une cellulase, une lipase, une oxydoréductase, et une glucosidase.

4. Méthode selon la revendication 1, dans laquelle le carbohydrate est choisi parmi le groupe constitué du glucose, saccharose, sirop de glucose, fructose, maltose, lactose, tréhalose, oligosaccharides, dextrines limites, dextrines, amidon, mannose, et galactose.

5. Méthode selon la revendication 1, dans laquelle le polyol est choisi parmi le groupe constitué de sorbitol, mannitol, érythrol, ribitol et xylitol.

6. Méthode selon la revendication 1, dans laquelle le carbohydrate et/ou le polyol est ajouté immédiatement après une étape de récupération utilisant une unité de concentration.

7. Méthode selon la revendication 1, dans laquelle le carbohydrate et/ou le polyol est ajouté immédiatement après ultrafiltration.

8. Méthode selon la revendication 1, dans laquelle le carbohydrate est ajouté dans une quantité de 0,1-75% (poids/poids).

9. Méthode selon la revendication 1, dans laquelle le polyol est ajouté dans une quantité de 0,1-80% (poids/poids).

10. Méthode selon la revendication 1, dans laquelle le polyol est un mélange de deux polyols.

11. Méthode selon la revendication 1, dans laquelle le pH est ajusté durant le processus de récupération.

12. Méthode selon la revendication 1, dans laquelle la température est ajustée durant le processus de récupération.

13. Méthode selon la revendication 1, dans laquelle des sels sont ajoutés durant le processus de récupération.

14. Méthode selon la revendication 13, dans laquelle les sels sont choisis parmi le groupe constitué de sels de magnésium, sodium, potassium, ammonium et calcium de chlorure, citrate, acétate, formiate et sulfate.
